Europäisches Patentamt

European Patent Office

Office européen des brevets

(11) Publication number: **0 299 706**
**A2**

(12) # EUROPEAN PATENT APPLICATION

(21) Application number: 88306334.9

(22) Date of filing: 12.07.88

(51) Int. Cl.⁴: **C12N 9/64 , C12N 9/70 , C12N 9/72 , C12N 15/00 , A61K 37/54**

(30) Priority: 13.07.87 US 72426
29.06.88 US 211279

(43) Date of publication of application:
18.01.89 Bulletin 89/03

(84) Designated Contracting States:
AT BE CH DE ES FR GB GR IT LI LU NL SE

(71) Applicant: **COLLABORATIVE RESEARCH INC.**
**Two Oak Park**
**Bedford Massachusetts 01730(US)**

(72) Inventor: **Baltimore, David**
**26 Reservior Street Cambridge**
**Massachusetts 01701(US)**
Inventor: **Moir, Donald T.**
**39 Virginia Road Waltham**
**Massachusetts 02154(US)**
Inventor: **Broeze, Robert J.**
**73 Agnes Drive Framingham**
**Massachusetts 01701(US)**

(74) Representative: **Jump, Timothy John Simon et al**
**VENNER, SHIPLEY & CO. 368 City Road**
**London EC1V 2QA(GB)**

(54) Nonglycosylated plasminogen activator and method of preparation.

(57) Novel, structurally modified plasminogen activators are provided which are incapable of receiving N-linked carbohydrate residues. The amino acid modified forms of the plasminogen activators retain their specific plasminogen activator activity and, thus, continue to function to activate plasminogen in initiating blood clot lysis.

EP 0 299 706 A2

## NONGLYCOSYLATED PLASMINOGEN ACTIVATOR AND METHOD OF PREPARATION

This invention relates to a nonglycosylated plasminogen activator and a method of preparing the same. Mammalian plasma contains an enzymatic system capable of dissolving the fibrin in blood clots. One component of this fibrinolytic system, plasminogen activator, generates the active enzyme plasmin by limited proteolysis of the zymogen plasminogen. Plasmin then degrades the fibrin network of a clot to form soluble products. Thus, there is therapeutic potential for the individual components of this system and especially for the plasminogen activators because they can control the initiation of the process.

There are two drugs commercially available for thrombolytic therapy which function as plasminogen activators: streptokinase, a bacterial protein, and urokinase, a serine protease isolated from human urine. Both of these plasminogen activators can activate plasminogen to form plasmin. Urokinase and streptokinase are employed in clinical practice today, in spite of their many inherent deficiencies. For urokinase, these are largely due to the very short (3-15 minutes) useful half-life of urokinase following its injection into humans. Furthermore, urokinase exists entirely in a form which is enzymatically fully active before injection, immediately upon injection, it reacts with the protease inhibitors that are normally present in relatively high concentrations in plasma and body fluids, and is thereby inactivated.

The use of streptokinase as a substitute for urokinase in such therapy is limited by the fact of its bacterial origin. Since streptokinase is a protein foreign to man, injection of streptokinase can in some cases give rise to the production of neutralizing antibodies which block its action and to allergic reactions that are harmful, and potentially fatal, to the recipient.

Thrombolysis with both of these substances, urokinase and streptokinase, however, is sometimes associated with systemic activation of plasminogen which can produce indiscriminate digestion of coagulation proteins, and significantly increase the risk of hemorrhage during treatment.

Plasminogen activators have been extracted from normal and tumor tissues and are produced by certain cells in culture. The plasminogen activators derived from these sources are serine proteases which have been classified into two major classes: urokinase-type plasminogen activators and tissue type plasminogen activators (TPA), based on differences in their immunological properties. Both types of enzymes are commonly isolated as proteins having two polypeptide chains linked by disulfides. However, it has been shown that under certain conditions both urokinase-type and tissue-type plasminogen activator can be isolated as single-chain enzymes which may be converted to the two chain form (see Rijken, D.C., Hoylaerts, M., and Collen, D. (1982) J. Biol Chem. 257: 2920-2925; Wun, T-C., Ossowski, L. and Reich, E. (1982) J. Biol. Chem. 257: 7262-7268; Nielsen, L.S., Hansen, J.G., Skriver, L., Wilson, E.L., Kaltoft, K., Zeuthen, J. and Dano, K. (1982) Biochemistry 21: 6410-6415; Husain, S.S., Gurewich, V and Lipinski, B (1983) Arch. Biochem. Biophys. 220: 31-38 and Kohno, T., Hopper, P. , Lillquist, J., Suddith, R.L., Greenlee, R. and Moir, D.T. (1984) Biotechnology 2: 628-634).

For example, a urokinase-type plasminogen activator, urinary urokinase, was originally isolated from fresh urine (Husain et al. 1983) supra), as a single chain molecule of about 50.000 molecular weight and subsequently from a human epidermoid carcinoma cell line HEp3 (Wun et al. (1982) supra), from human glioblastoma cells (Nielsen et al. (1982) supra) and from a permanent human kidney cell line (Kohno et al. (1984) supra). This single chain urokinase-type plasminogen activator, frequently termed prourokinase (PUK) or single chain urinary plasminogen activator (SCUPA), consists of a single polypeptide chain 411 amino acid residues long. The single chain urokinase-type plasminogen activator is converted to an active form by incubation with plasmin. The two chain form of the enzyme, also known as high molecular weight (HMW) urokinase, has been reported to have a molecular weight in the range from 47,000 to 56,000 and appears to give rise, upon reduction, to two chains of about 31.000 to 34,000 molecular weight and about 20,000 molecular weight (see Husain et al. (1983) supra). The single chain form is converted to HMW urokinase by the proteolytic cleavage of the peptide bond between lysine[158] and isoleucine[159]. An active low molecular weight (LMW) form of urokinase of about 31,000 molecular weight has also been characterized and is apparently derived from the larger chain of HMW urokinase.

The production of prourokinase from natural sources, the conditioned medium of human cells cultured in vitro, provides sufficient quantities of material to determine its usefulness in the treatment of myocardial infarctions, heart attacks. This type of prourokinase production should also provide appropriate material to determine its usefulness in the treatment of pulmonary embolism, deep vein thrombosis, and strokes. Larger quantities of prourokinase material will be needed to meet expected pharmaceutical demands, and the economics of such production will play a key role in making prourokinase a widely-used pharmaceutical.

One possible approach to address the economics of PUK production would be to apply recombinant DNA techniques to engineer production by yeast (Duncan, M.J., Stashenko, K. and Raina, J. (1986) SIM News, 1986 Society for Industrial Microbiology Annual Meeting, 36 No. 4: A-14) or by mammalian cell culture which was the approach used for obtaining larger quantities of TPA. In order to circumvent possible misfolding of a protein, investigators have sought to secrete proteins from the cell since it is this process which produces properly folded proteins. However, it is in the secretion pathway of yeast, other fungi and mammalian cells that protein glycosylation is known to occur and frequently the secreted proteins have heterologous glycosylation patterns which are potentially immunogenic. See Parekh, R.B., Tse, A.G.D., Dwek, R.A., Williams, A F. and Rademacher, T.W. (1987) The EMBO Journal 6: 1233-1244, Dijkmans, R., Heremans, H. and Billiau, A. (1987) J. Biol Chem. 262: 2528-2535 and Jabbar, M.A. and Nayak, D.P. (1987) Molec. Cell. Biol. 7: 1476-1485.

Initially, a more extensive investigation of human TPA as a potential thrombolytic agent was hampered by its extremely low concentration in blood, tissue extracts, vessel perfusates and cell culture. The production of TPA in cultured mammalian cells using recombinant DNA techniques provides sufficient quantities of material to allow the determination of extent and optimum conditions for usefulness in the treatment of pulmonary embolism, deep vein thrombosis, heart attacks and strokes. The mammalian cell lines developed, Chinese hamster ovary (CHO) cells, secrete the TPA into the cellular medium.

The recombinant-derived tissue-type plasminogen activator is indistinguishable in amino acid composition from the tissue plasminogen activator isolated from normal human tissue and from human melanoma cells cultured in vitro. However, it is glycosylated in the host CHO cells as it is secreted, and the pattern of carbohydrate applied may vary with culture conditions and host cell used. This possibility of variation in carbohydrate pattern is a potential problem.

Although it catalyzes the proteolytic activation of plasminogen in much the same way as does urokinase, TPA differs from urokinase in a number of important respects. The two enzymes are chemically dissimilar. They have different molecular weights and each fails to react with antibodies to the other enzyme. The catalytic action of TPA is enhanced by fibrin whereas that of urokinase is not. Not unlike urokinase-type plasminogen activator, TPA occurs in two different forms, one being a single chain form which is converted by plasmin or trypsin into two chains linked by disulfide bridges, which are considered to be a degradation product of the former. The two chain form is thought to be fibrinolytically more efficient in fibrinolytic test systems (in vitro) than the one chain form. Aprotinin has been shown to inhibit conversion of the one chain form into the two chain form so that the one chain form may be recovered as the only predominating form. Yet both forms are considered to be tissue plasminogen activators. (For review of this subject matter see Rijken et al (1982) supra and Vehar, G.A., Kohr, W.J., Bennett, W.F., Pennica, D., Ward, C.A., Harkins, R.N. and Collen, D. (1984) Biotechnology 2: 1051-1057.

In spite of the application of recombinant DNA techniques to mammalian cell cultures systems, significant economic and therapeutic benefits are to be gained by producing a plasminogen activator molecule in non-mammalian cell culture systems via recombinant DNA techniques that affords the production of a molecule which will neither elicit the production of neutralizing antibodies nor allergic reactions and yet have the capability of being produced in large, economic quantities. None of these advantages appear to apply to any plasminogen activator molecule but the newly isolated nonglycosylated plasminogen activator molecule of the present invention.

It is an object of the present invention to provide a modified plasminogen activator having no site for glycosylation.

A further object of the present invention is to provide a modified plasminogen activator having no site for N linked glycosylation utilized in yeast and mammalian cells in accordance with the preceding object with an amino acid change in the asparagine-linked carbohydrate receiving region.

A further object of the present invention is to provide means and methods of producing the modified plasminogen activator of the preceding objects.

Another object of the present invention is to demonstrate that the modified plasminogen activator product expressed by a host organism transformed by recombinant means is processed to biologically active plasminogen activator within the host and then transported across the cell's membrane.

Another object is to provide a nonglycosylated secreted, plasminogen activator.

The modified plasminogen activator, according to the present invention, is provided by recombinant DNA techniques with an amino acid modification such that an amino acid change in the asparagine-linked carbohydrate receiving region, i.e. the amino acid sequence asparagine-X-threonine/serine, prevents the addition of N-linked carbohydrate and a polypeptide is produced and maintained as a nonglycosylated protein.

A method is provided for increasing the therapeutic utility of plasminogen activators without substantially decreasing fibrinolytic activity, which comprises modifying the asparagine and/or threonine (serine) residues of the amino acid consensus sequence found within the amino acid sequence of plasminogen activators. The method can comprise substitution, insertion or deletion.

The nonglycosylated plasminogen activator of this invention can be injected into blood in the body in vivo at a dosage level sufficient to dissolve clots without causing unwanted body change. Typical parameters of use for myocardial infarction can be as heretofor known for streptokinase.

It is an unprecedented feature of this invention that plasminogen activator is provided in a non-glycosylated form which can be particularly effective in dissolving blood clots without eliciting any type of allergic reaction thought possible for some of the previously known plasminogen activators. The modified plasminogen activator is electrophoretically a single, homogenous protein, structurally similar to its unmodified counterpart but distinguishable in that it contains an amino acid modification at the asparagine and/or threonine (serine) residues of the amino acid consensus sequence found within the amino acid sequence of plasminogen activators.

It is a further feature of the invention that modification of the plasminogen activator amino acid consensus coding sequence by site-directed mutagenesis or other means, results in nonglycosylated plasminogen activator which increases its value for therapeutic use over that of unmodified plasminogen activator.

The nonglycosylated plasminogen activator of this invention can be made by conventional genetic engineering techniques using yeast, other fungi, mammalian or other known host cells, or by other techniques.


## BRIEF DESCRIPTION OF THE DRAWINGS


FIG. 1 depicts the nucleotide sequence of prourokinase and its amino acid sequence. The amino acids for mature prourokinase are numbered from 1 to 411. The arrow indicates the start of the initial isolated clone;

FIG. 2 is a schematic representation of probes used in screening the phage library for prourokinase;

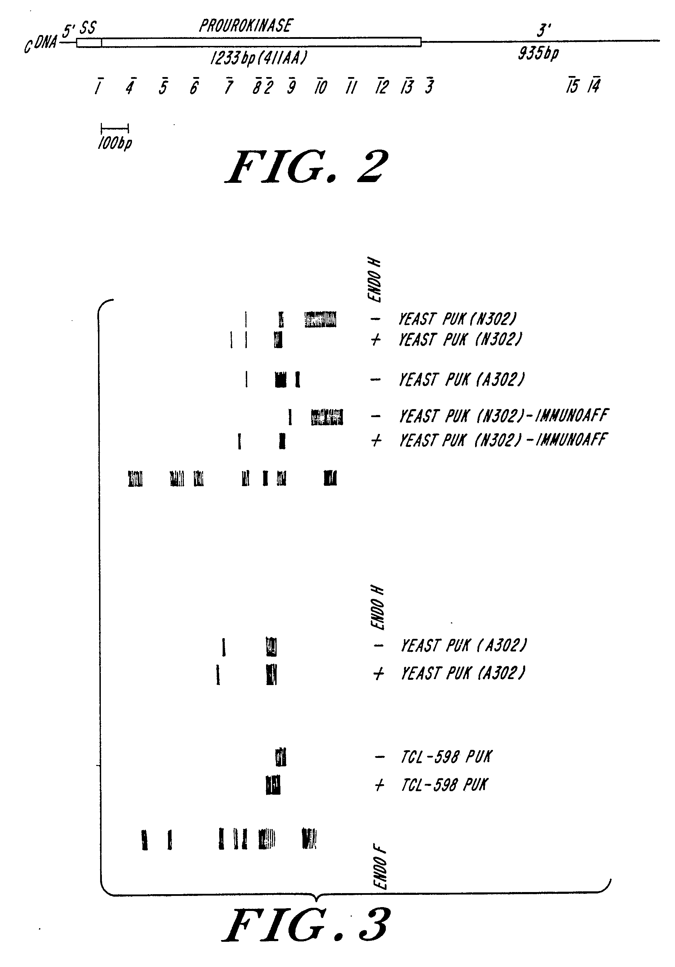

FIG. 3 is a representation of the silver stained gel to show the production of nonglycosylated prourokinase [Yeast PUK (N302) is PUK secreted by the strain carrying the plasmid pCGS715 (TPI-promoted invertase secretion signal PUK with asparagine at position 302); Yeast PUK (A302) is PUK secreted by the strain carrying the plasmid pCGS721 (TPI-promoted invertase secretion signal PUK with alanine at position 302); TCL-598 PUK is PUK isolated from a permanent human kidney cell line (Kohno et al. (1984) supra); all above PUKs with (+) and without (-) treatment by Endo H (Endoglycosidase H) and Endo F (Endoglycosidase F)];

FIG. 4 is a schematic drawing of plasmids pCGS732 and pCGS740; and

FIG. 5 is a schematic drawing of plasmid pCGM16.


## DESCRIPTION OF PREFERRED EMBODIMENTS


The plasminogen activator to be modified in accordance with this invention may be any plasminogen activator which has an asparagine-linked carbohydrate receiving region, i.e. asparagine-X-threonine/serine, which results in N-linked carbohydrate addition. Thus, glycosylation as used herein is defined as the addition of N-linked carbohydrate to an appropriate amino acid.

Glycosylation of eukaryotic proteins may occur at the tripeptide sequences asparagine-X-threonine and asparagine-X-serine, where X may be any amino acid residue except possibly aspartic acid or proline (see Taussig, R. and Carlson. M. (1983) Nucleic Acids Research 11: 1943-1954). Such glycosylation is known in the art and further defined in Struck, D.K. and Lennarz, W.J. (1980) in The Biochemistry of Glycoproteins and Proteoglycans, Lennarz, W.J. Ed., Plenum Press, New York, 35-83, and Hubbard, S.C. and Ivatt, R.J. (1981) Ann. Rev. Biochem. 50: 555-583. It is at these specific asparagine residues that carbohydrate is attached creating N-linked glycosylated proteins. It is the inability to add such carbohydrates which is the feature of the present invention.

The plasminogen activator can be streptokinase, urokinase-type plasminogen activators, or tissue-type plasminogen activators; or any of the various molecular weight forms of the urokinase-type or tissue-type plasminogen activators; or any of the various single-chain or two-chain forms of these plasminogen activators. The molecular weights of the plasminogen activators can range from a molecular weight of about 31,000 daltons for low molecular weight urokinase to about 68,000 daltons for tissue-type plasminogen

4

activator or even lower or higher molecular weight plasminogen activators can be modified. The present invention can be practiced on any plasminogen activator which has an asparagine-linked carbohydrate receiving region.

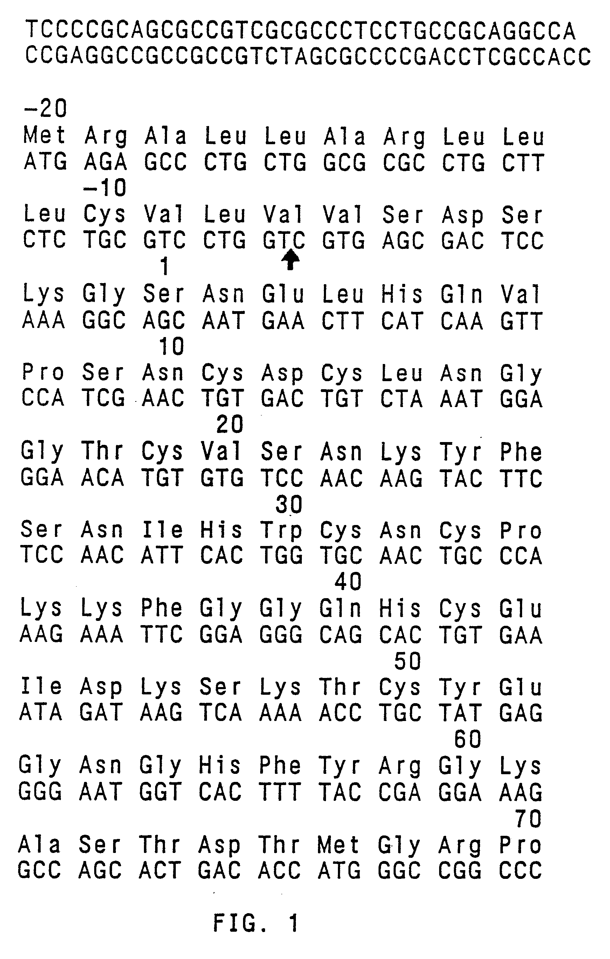

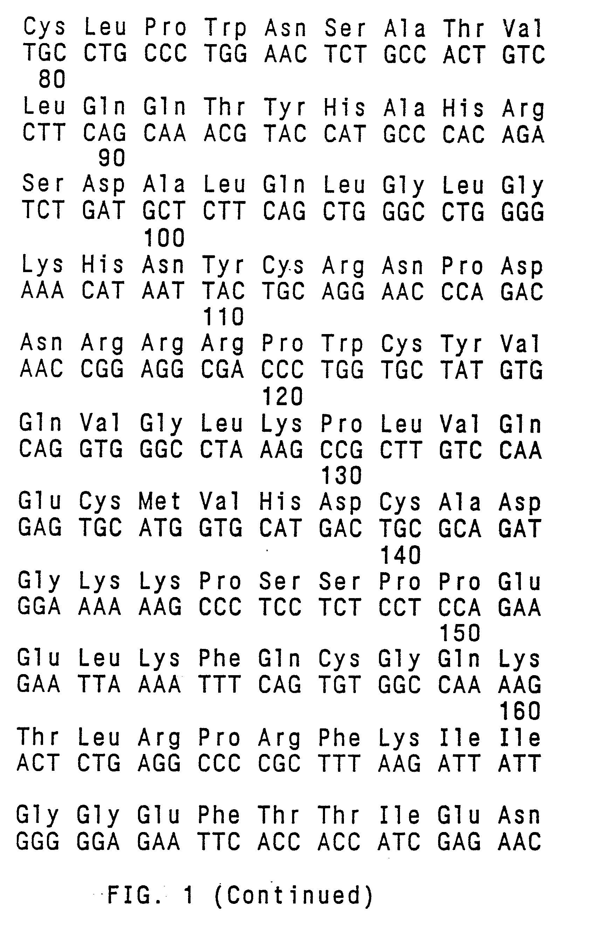

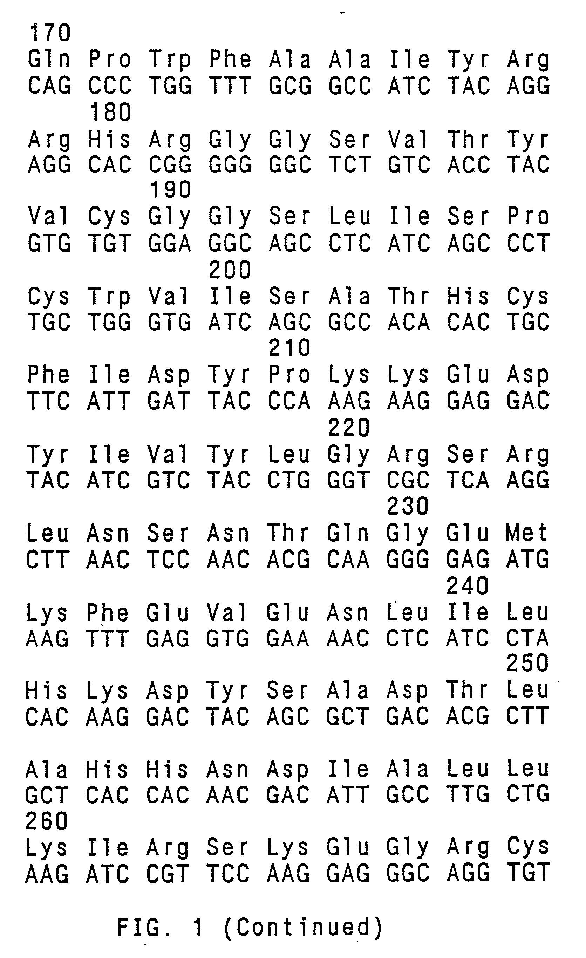

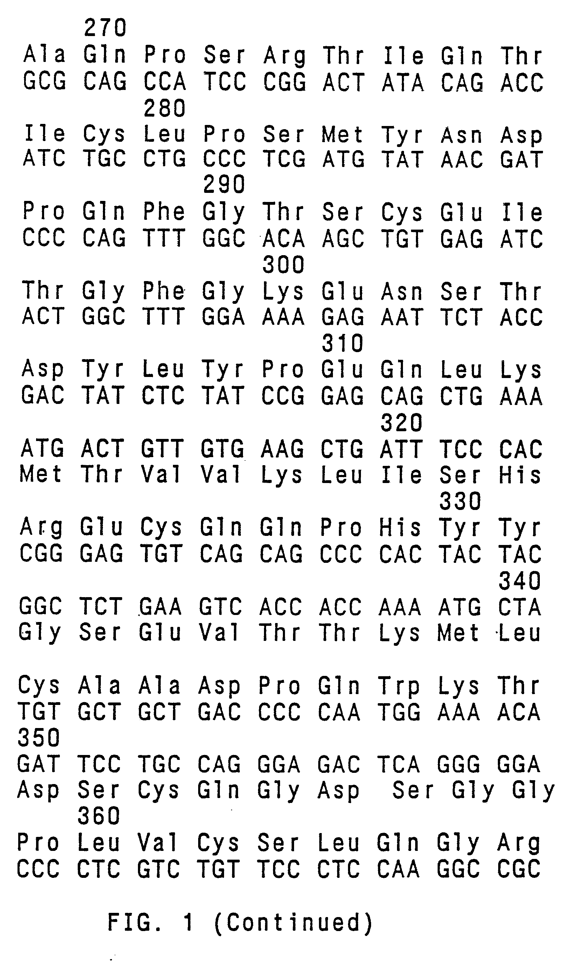

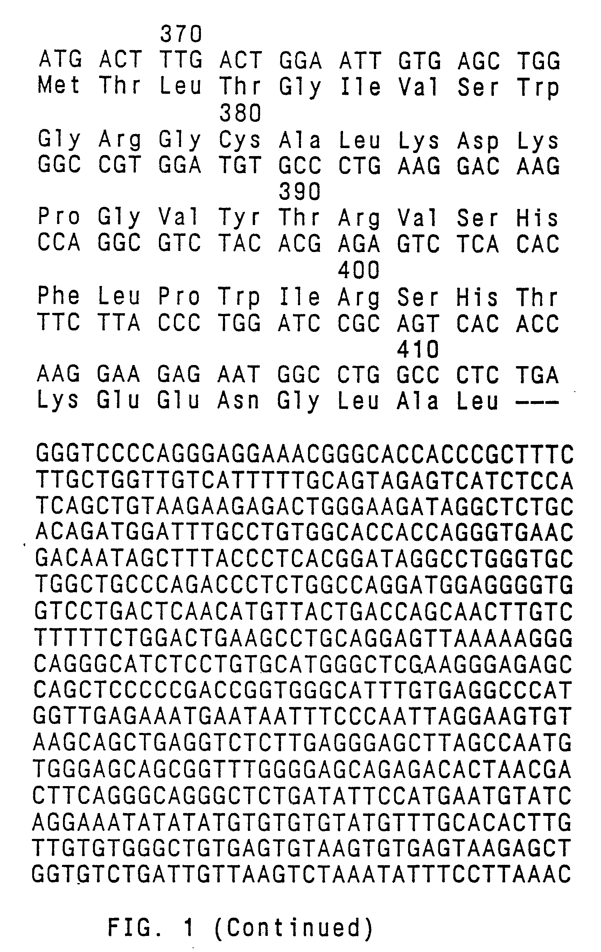

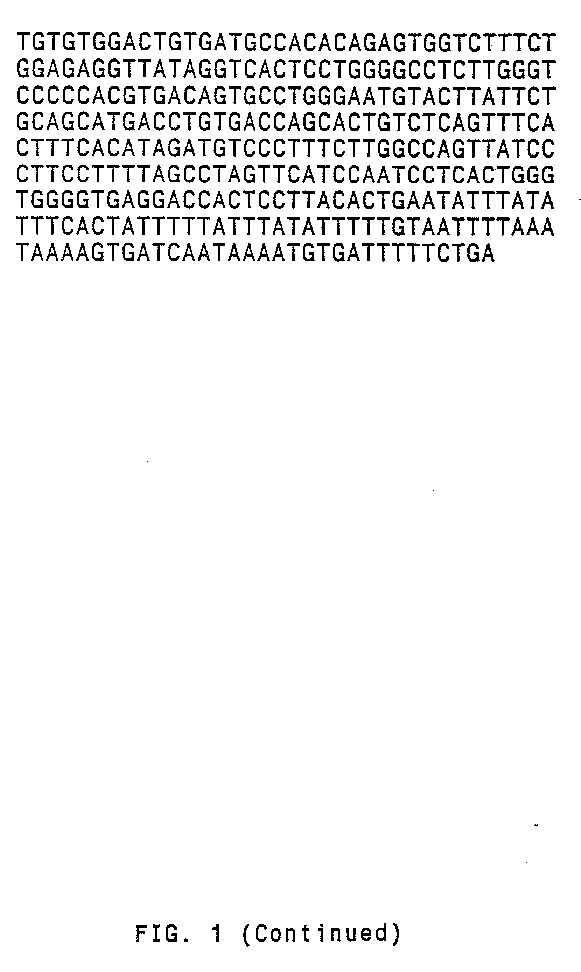

In the specific examples of this invention, prourokinase is obtained from human kidney cells (Kohno, T., Hopper, P., Lillquist, J.S., Suddith, R.L., Greenlee, R. and Moir, D.T. (1984) Biotechnology 2: 628-634). Prourokinase is defined as that plasminogen activator having a single polypeptide chain and which exhibits plasminogen activator activity expressed in terms of CTA (Committee on Thrombolytic Agents) units as determined by a fibrin plate assay as described by Brakman, P. (1967) in Fibrinolysis, Schelthema Holkema, Amsterdam, 1-124 and substantially as described in FIG. 1. The cells are grown to confluency in Dulbecco's Modified Eagles medium supplemented with 5% heat-inactivated NuSerum (Collaborative Research, Inc., Bedford, Massachusetts). Confluent cells are harvested by centrifugation after treatment with 0.25 percent trypsin for 15 minutes at 37°C and are frozen in liquid nitrogen.

The poly (A) RNA is isolated according to the method of Maniatis, T., Fritsch, E. F. and Sambrook, J. (1982) "Molecular Cloning. A Laboratory Manual", Cold Spring Harbor Laboratory, Cold Spring Harbor, New York, 191-198. Briefly, 5 grams of frozen cells are lysed with NP-40 and the nuclei are removed by ultracentrifugation. The cytoplasmic fractions is treated with proteinase K and the protein is removed by repeated phenol/chloroform:isoamyl alcohol extractions. The total cytoplasmic RNA is recovered by precipitation with ethanol with a yield of about 16 milligrams. The poly (A) RNA is isolated by oligo (dT) cellulose chromatography with a yield of about 200 micrograms. The intactness of the isolated poly (A) RNA is verified by in vitro translation in a rabbit reticulocyte lysate system. Conversion of poly (A) mRNA to double stranded cDNA is by standard procedures (see Wickens, M. P., Buell, G. N. and Schimke, R. T. (1978) J. Biol. Chem. 253: 2483-2495) using oligo (dT) as the primer of the first strand by reverse transcriptase, DNA polymerase I to synthesize the second strand, nuclease S1 treatment to insure that the ends are flush and chromatography over Biogel A150M for size selection.

The size-selected double-stranded cDNA is ligated to HindIII synthetic oligonucleotide linkers (Collaborative Research, Inc., Bedford, MA) and then is transformed into a single-stranded f1 phage vector (Zinder, N. D. and Boeke, J.D. (1982) Gene 19: 1-10; Bowden, D. W., Mao, J., Gill, T., Hsiao, K., Lillquist, J. S., Testa, D. and Vovis, G. F. (1984) Gene 27: 87-99).

Fifteen oligonucleotide probes, each 14 bases in length and homologous to portions of the PUK coding region, are synthesized by the automated phosphoramidite procedure (Applied Biosystems automated DNA synthesizer). The probes (number 1 to 15) are shown schematically in FIG. 2 together with their location within the gene.

For screening of the phage cDNA library, probes are mixed, labeled with $^{32}P$ by means of polynucleotide kinase and gamma-$^{32}P$-ATP and isolated from the residual label by means of a polyacrylamide gel. The resultant labeled probes are used to probe nitrocellulose plaque lifts from the library essentially according to the method of Benton, W. D. and Davis, R. W. (1977) Science 196: 180-182. The use of multiple probes insures that unexpected occasional polymorphisms within the gene or poor hybridization by certain probes due to unpredictable structure or sequence context problems does not prevent identification of the clones containing PUK. In this manner, approximately 50,000 clones are screened for prourokinase sequences. One of the colonies shows hybridization with eleven of the probes. Dideoxy-sequencing and restriction analysis of the latter clone named CGF31 shows that the clone contains a 2.2 kilobase insert and starts in the middle of the signal sequence (SS) (see FIG. 1). Further screening using a probe complementary to a region of the signal sequence reveals a second clone which harbors the missing part of the signal sequence.

The two inserts are subcloned into an expression vector at a BglII restriction site which is common to both inserts. Restriction analysis is carried out on the resultant subclone named pCGE195 and the latter cDNA is shown to encode full length prourokinase.

EXAMPLE 1

In order to construct a plasmid containing PUK cDNA with an amino acid modified N-linked carbohydrate consensus sequence such that nonglycosylated PUK is produced, a scheme is followed whereby the asparagine contained within the tripeptide consensus sequence, i.e. asparagine-X-threonine and/or asparagine-X-serine, is replaced with an amino acid other than asparagine. In this specific example using PUK, only one tripeptide consensus sequence is available for N-linked carbohydrate addition so that the asparagine found at amino acid position numbered 302 of the sequence asparagine (position 302)-serine-threonine of FIG. 1 is replaced by alanine. By changing the asparagine residue at position 302 of PUK into a codon for alanine, the site of N-linked glycosylation utilized in both yeast and mammalian cells is lost. In order to accomplish this, a site-directed mutagenesis procedure involving priming synthesis of the mutant gene from a single-stranded template by means of a synthetic oligonucleotide with a mismatch at the asparagine 302 codon is utilized. The template is most of the normal PUK cDNA gene cloned into the single-stranded phage f1. The resulting double-stranded f1 molecules containing a mismatch at the asparagine 302 codon of PUK are transformed into E. coli and plaques are probed with the labeled mismatch primer to identify those containing the modified or mutant PUK gene. A portion of that mutant gene is spliced into a yeast expression vector. Yeast cells carrying this mutant gene produce active PUK and secrete it as efficiently as the normal glycosylated version. Purification of the secreted PUK and size analysis of the secreted PUK by polyacrylamide gel electrophoresis confirms that the resulting amino acid modified secreted PUK bears no N-linked carbohydrate.

Briefly, two synthetic oligonucleotide primers are synthesized as described previously: one primer is complementary to codons 299-305, 5´ GGA AAA GAG GCC [codon 302] TCT ACC GAC 3´, except that GCC is the codon for alanine and replaces AAT for asparagine normally found at that position, and the other primer is complementary to codons 16-21, 5´ GGA GGA ACA TGT GTG TCC 3´, which primer is the "helper" to insure that the full second strand of the cDNA is produced in the phage and that nucleases will not damage the former primer. The aforementioned synthetic oligonucleotide primers are labeled with $^{32}$P by means of polynucleotide kinase and gamma-$^{32}$P-ATP and isolated from the residual label by means of a polyacrylamide gel. The kinased oligonucleotide primers are annealed to the single stranded template DNA of phage designated CGF31. The single-stranded DNA of f1 phage, designated CGF31, is constructed such that it contains most of the antisense strand of the PUK cDNA gene. The annealed oligonucleotide primers are extended with DNA polymerase I (Klenow fragment) and the resulting double-stranded molecule is ligated closed.

An E. coli strain designated CGE6 is transformed with the DNA and a strain designated CGE5 is added as transfection host. Transformation is performed in this manner because strain CGE6 transforms more efficiently than strain CGE5, but strain CGE6 lacks pili and cannot be reinfected with f1 phage By plating the transformed CGE6 along with CGE5, the CGE5 cells serve as host for infection by the f1 phage and generation of plaques.

The plaques are plated out and nitrocellulose "lifts" of the plaques are made. The "lifts" are probed with the kinased primer which is complementary to codons 299-305, 5´ GGA AAA GAG GCC [codon 302] TCT ACC GAC 3´, except that GCC is the codon for alanine and replaces AAT for asparagine, in order to distinguish those phage which contain the mutant PUK gene from those with the wild-type PUK gene. The nitrocellulose is washed and exposed to x-ray film and then rewashed at higher temperature and re-exposed such that a clear distinction between mutant plaques and wild-type plaques are noted.

The plaques are "purified" by re-plating the picked mutant phage and re-screened to insure that the picked plaque is not mixed with phage carrying the wild-type PUK gene. Dideoxy sequencing of the mutant PUK gene in the f1 phage confirms the replacement of the asparagine at position 302 with alanine. Restriction endonuclease analysis of one transformant· verifies the creation of the mutant PUK gene by showing the acquisition of a new StuI site as predicted such that alanine is now found at position 302.

the mutated PUK gene is placed into a yeast expression vector by methods known to those skilled in the art. A 931 bp BglII to BamHI fragment from the mutant RFI DNA, described above, is removed and placed into a vector, designated pCGS715, in place of that portion of the wild-type PUK cDNA gene, to generate vector pCGS721.

Vector pCGS715 is basically pCGS128 as described if Goff, C.G , Moir, D.T., Kohno, T., Gravius, T.C., Smith, R.A., Yamasaki, E. and Taunton-Rigby, A. (1984) Gene 27: 35-46. The triose phosphate isomerase (TPI) promoted invertase secretion signal fusion to prourokinase is placed between EcoRI and HindIII where formerly the GAL1 promoted prochymosin gene resided. The TPI promoted invertase secretion signal is the promoter region from the yeast TPI gene (see Alber, T. and Kawasaki, G. (1982) J. Mol. Appl. Genet. 1: 419-434) from the BglII site approximately 800 basepairs 5´ from the translation initiation ATG codon of the TPI coding region to the C residue which lies 18 basepairs 5´ to the TPI ATG codon. A pair of oligonucleotides, 13 bases in length (TATAAGCTCCATG and CATGGAGCTTATA), synthesized by the automated phosphoramidite procedure (Applied Biosystems automated DNA synthesizer), connects the C residue of the TPI coding region to a 57 basepair fragment of the invertase secretion signal. The 57 basepair fragment of the invertase secretion signal extends from basepair +1 to +57 in the sequence as described in Taussig, R. and Carlson, M. (1983) Nucleic Acid Res. 11: 1943 1954 (see Figure 2 in the cited reference). Finally, the portion of the PUK cDNA encoding mature PUK as shown in FIG. 1 (nucleotides corresponding to amino acids labeled 1 to 411) is fused directly to nucleotide +57 of the invertase secretion signal.

The construction of plasmid pCGS721 containing the alanine codon at position 302 in place of the asparagine codon in pCGS715 is verified by restriction endonuclease analysis which shows the loss of an EcoRI site due to the asparagine 302 codon change.

Large-scale plasmid preparations of the constructions pCGS721 (TPI-promoted invertase secretion signal PUK with alanine at position 302 on an autonomously replicating yeast/E. coli shuttle vector) and pCGS715 (TPI-promoted invertase secretion signal PUK with asparagine at position 302 on an autonomously replicating yeast/E. coli shuttle vector) are made. Both plasmids, pCGS721 and pCGS715, are transformed into yeast strain CGY1585 (mato ura3-52 leu2-3,112 ssc1 ssc2). The yeast transformants are grown in semi-synthetic medium and the PUK activity found in the broth is measured by means of the fibrin plate assay. The strain CGY1585/pCGS721 produces about 3500 units/g dry cells of PUK activity in the culture broth. The secreted PUK of strain CGY1585/pCGS721 is partially purified from the culture broth, applied to an SDS-polyacrylamide gel and analyzed by silver staining (see FIG. 3) and immunoblot. The band corresponding to amino acid modified PUK (mutant PUK) prepared both from extracts and broth is found to migrate faster that non-mutant PUK produced in the same yeast host strain. This result is consistent with the absence of carbohydrate on the mutant PUK.

Similar results were also obtained by examining unfractionated yeast broth from CGY1585 cells carrying plasmid pCGS721 or pCGS715 by means of the immunoblot method of Towbin, H., Staehelin, T., & Gordon, J. (1979, Proc. Natl. Acad. Sci. USA 76: 4350-4354). In that case, the antibody used to visualize the PUK was made in rabbits against human urokinase isolated from TCL-598 cells (Kohno et al. , 1984, supra), and visualization was by means of $^{125}$I-labeled protein A (NEN Research Products, Boston, MA) and autoradiography. In the following examples, PUK with an alanine codon at position 302 is denoted as PUK(A302), with an asparagine codon at 302 as PUK(N302), with a glutamine codon at 302 as PUK(Q302), etc., following the single letter code for amino acids. Yeast-produced PUK(N302) from CGY1585 carrying plasmid pCGS715, yeast-produced PUK(A302) from CGY1585 carrying plasmid pCGS721, and TCL-598 produced PUK from a human kidney cell line (Kohno et al., 1984, supra) are shown in FIG. 3 either treated or not treated with endoglycosidases H or F (NEN Research Products, Boston, MA) which remove carbohydrate chains from proteins glycosylated by yeast or mammalian cells, respectively.

The shift in electrophoretic mobility (ie, apparent molecular weight) following treatment to remove the carbohydrate is an indication of the amount of carbohydrate applied to the PUK species shown. PUK secreted from mammalian cells ("TCL-598 PUK" labeled lanes) undergoes only a small shift in mobility after treatment with endoglycosidase F consistent with the removal of a single "complex" type carbohydrate chain from asparagine 302 (Gunzler, W., Steffen, G., Otting, F., et al., 1982, Hoppe-Seyler's Z. Physiol. Chem. 363: 1155-1165). In contrast, glycosylated PUK secreted from yeast cells ("Yeast PUK (N302)" labeled lanes) undergoes a large mobility shift after treatment with endoglycosidase H, consistent with the known tendency of the yeast cell to heavily glycosylate its own glycoproteins such as invertase (Trimble, R.B. and Maley, F., 1977, J. Biol. Chem. 252: 4409-4412). It is clear from FIG. 3 that modified PUK containing alanine at position 302 receives no detectable carbohydrate when it is secreted from yeast because there is no significant change in its mobility after treatment with endoglycosidase H. Thus, the alteration of the codon 302 for asparagine to a codon for alanine in the PUK cDNA gene results in secretion of an apparently non-glycosylated but fibrinolytically active PUK protein from yeast cells.

## EXAMPLE 2

In another example of this invention, a yeast strain secreting high levels of non-glycosylated active PUK into the fermentation broth was constructed. As described previously, secretion of mammalian proteins from Saccharomyces cerevisiae may be aided by several factors, including the addition of a yeast secretion signal, use of host strains carrying specific mutations called "supersecreting" (ssc1 and ssc2), and integration of the transcriptional unit (ie, promoter + secretion signal + cDNA gene) into the yeast chromosomes (see Smith, R.A., Duncan, M.J., and Moir, D.T., 1985, Science 229: 1219-1224). The first two elements were incorporated into EXAMPLE 1 above; the last element, integration of the transcriptional unit, is incorporated according to the methods in this EXAMPLE to provide a yeast strain secreting higher levels of PUK into the culture broth.

Two types of yeast integrating vector were constructed -- one based on plasmid YIp5 (Struhl, K, Stinchcomb, D.T., Scherer, S., and Davis, R.W., 1979, Proc. Natl. Acad. Sci. USA 76: 1045-1039) and one based a yeast Ty transposable element (Cameron, J.R., Loh, E.Y., and Davis, R.W., 1979, Cell 16: 739-751). In order to construct the first vector, Ylp5 was modified in the following manner. First, Ylp5 DNA was cut with restriction endonuclease NcoI within the URA3 gene and a DNA fragment encoding a functional LEU2 gene and having NcoI ends was added by ligation with T4 DNA ligase. The DNA encoding LEU2 was obtained from plasmid pJS34 (D. Fraenkel) Harvard Medical School, Department of Microbiology, Boston, MA), but it can also be constructed by isolating the approximately 2.2 kb DNA fragment from plasmid pYT11-LEU2 (Cohen, J.D., Eccleshall, T.R., Needleman, R.B., Federoff, H., Buchferer, B.A. , & Marmur, J., 1980, Proc. Natl. Acad. Sci. USA 77: 1078-1082) obtained after restriction with endonucleases XhoI and SalI, filling out the overhanging termini with E. coli DNA polymerase I (Klenow fragment) in the presence of all four dNTPs, and ligating NcoI oligonucleotide linkers in place according to standard methods.

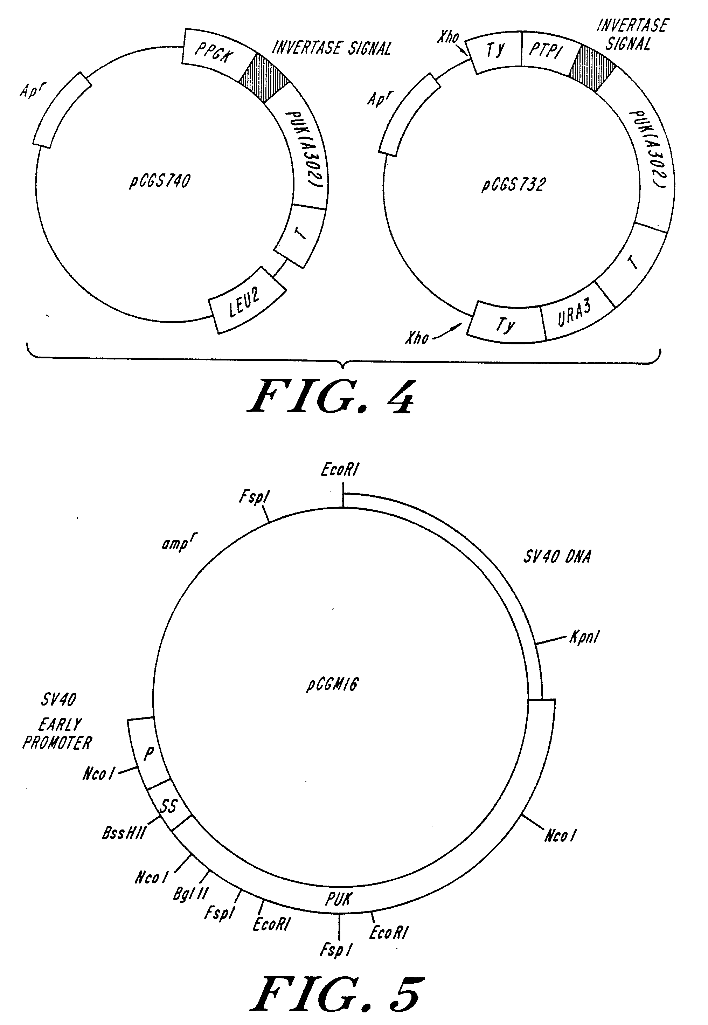

Second, a PGK-promoted invertase signal sequence fusion to PUK(A302)followed by the transcriptional terminator region from SUC2 was added between the EcoRI site and the disrupted URA3 gene described above . Access to the 3 end of the PGK promoter (Kawasaki, G. and Fraenkel, D.G., 1982, Biochem. Biophys. Res. Communic. 108: 1107-1112) was obtained by Bal31 digestion from the SacI site in the codon for amino acid residue 154 followed by ligation of NcoI (ACCATGGT) linkers. Fusion to the invertase secretion signal was accomplished with a synthetic oligodeoxy-nucleotide pair, 5'CATGATGCTTTTGCA3' and 5'AGCTTGCAAAAGCAT3', bridging the gap between the NcoI site of the PGK promoter region and the HindIII site within the invertase secretion signal coding region (Taussig, R. and Carlson, M., 1983. Nucl. Acids Res. 11: 1943-1954). The remainder of the invertase secretion signal coding DNA together with the entire PUK(A302) cDNA and the SUC2 transcriptional terminator DNA sequence was obtained as an approximately 3.2 kb HindIII to SalI fragment from plasmid pCGS721. The resulting plasmid pCGS740 (FIG. 4) provides a PGK-promoted invertase signal sequence fusion to PUK(A302) whose presence can be selected by demanding LEU2 function. According to the method of Orr-Weaver, T.L., Szostak. J.W., and Rothstein, R.J. (1983, Methods in Enzymol. 101: 228-245) integration can be directed to the PGK or the LEU2 yeast genes on the chromosomes by restriction of the plasmid DNA with ClaI, which will cut in either of those plasmid DNA sequences, and transformation of the cut DNA into yeast cells.

The second integrating plasmid provides a TPI-promoted invertase secretion signal fusion to PUK(A302) which can be selected by demanding URA3 function. DNA homology for integration is provided by the yeast transposable element Ty (Cameron et al., 1979, supra). A variety of Ty elements have been cloned from S. cerevisiae (Clare, J. and Farabaugh, P.J., 1985, Proc. Natl. Acad. Sci. USA 82: 2829-2833; Hauber. J., Nelbock-Hochstetter, P., and Feldman, H., 1985, Nucl. Acids. Res. 13: 2745-2758), and any of them will suffice for this application since they only serve to provide homology for integration of the PUK transcriptional unit into other Ty elements lodged in the chromosomes of the desired yeast host strain. In this case, a particular cloned yeast Ty element, TyH25, was obtained from J. Boeke (Johns Hopkins University School of Medicine, Baltimore, MD) as a BamHI linkered insert in pBR322 termed plasmid pJEF648.

Plasmid pJEF648 was modified in the following way. One of the PstI sites, located about 1.1 kb from the XhoI site found in one of the terminal delta elements, was converted to a unique XbaI site by using synthetic XbaI oligonucleotide linkers (Collaborative Research, Inc., Bedford, MA). Next, about 2.8 kb of internal Ty sequence was removed from the vector by complete digestion with XbaI and partial digestion with EcoRI (desired site is the EcoRI site about 1.7 kb from the XhoI site in the opposite terminal delta). This was replaced with an approximately 4.0 kb EcoRI to SalI(following addition of XbaI linkers) DNA fragment from pCGS721 containing the yeast TPI promoter, the invertase secretion signal fusion to PUK-(A302) and the transcriptional terminator region from the yeast SUC2 gene. Finally, an XbaI linkered 1.1 kb URA3 gene was inserted into the unique XbaI site to yield plasmid pCGS732 (FIG. 4). Complete digestion with XhoI yields an approximately 6.7 kb fragment containing the PUK(A302) transcriptional unit surrounded by at least 1 kb of Ty sequence homology on each side. Transformation of yeast strains with this XhoI digest results in integration into chromosomal Ty elements by homologous replacement of native Ty sequences with the DNA encoding a TPI-promoted invertase secretion signal fusion to PUK(A302) (Rothstein, R.J., 1983, Methods in Enzymol. 101: 202-211).

A yeast strain secreting high levels of PUK(A302) was constructed by using these two integrating plasmids as follows. Host strain CGY1465 (MAT alpha ura3-52 leu2-3,112 ssc1-1) was transformed first to leucine prototrophy with ClaI digested pCGS740 and then to uracil prototrophy with XhoI digested pCGS732. Host strain CGY1468 (MATa ura3-52 leu2-3,113 pep4-3 ssc2-1) was transformed to uracil prototrophy with XhoI digested pCGS732. Since these conditions would permit the integration of multiple tandem copies of pCGS740 into either the PGK and/or the LEU2 locus and would permit multiple replacements into any of some 30 Ty loci, the resulting transformants were screened for secretion levels of PUK(A302) by using the fibrin plate assay for culture broths. The most productive CGY1465 and CGY1468 transformants were crossed together, sporulated and dissected. Meiotic progeny secreting the most PUK-(A302) activity were analyzed for the presence of integrated PUK(A302) cDNA copies by Southern blot and for the presence of the ssc1 and ssc2 mutations by crossing to ssc1 and ssc2 tester strains of opposite mating type and determining the competency of the resulting diploids to sporulate. Homozygous ssc1 or ssc2 diploids do not sporulate efficiently (Smith et al., 1985, supra). One progeny spore designated CGY1892 was judged to carry both the ssc1-1 and ssc2-1 mutations and to contain pCGS740 integrated into the LEU2 locus and pCGS732 integrated into a Ty locus.

Yeast strain CGY1892 was maintained in frozen ampules in 40% glycerol at -70° C until use. Thawed cells were inoculated into 100 mls of SM-IV medium (described below) at 0.5 to 1.0% inoculum densitites in baffled 500 ml shake flasks agitated at 200 RPM at 25° C. In late log phase, between 2,000 and 3,000 Klett units, cultures were transferred to a 20 liter fermentor at 1-1.5% inoculum levels. SM-IV medium was used for all fermentations, with constant agitation of 1,000 RPM with a Chemap "Effigas" agitator and 2-3 VVM airflow. Fed batch fermentation was conducted by periodic addition of glucose as indicated by the sudden increase in dissolved oxygen concentration associated with depletion of glucose and ethanol secondary carbon sources. Glucose feeding was accomplished by removing 15% of the broth volume and refeeding with an equal volume of 500 g/l glucose. Initial medium pH of 6.35 decreased during the run to 6.00 where it was maintained by periodic addition of 50% NaOH.

SM-IV medium compostion was as follows, with amounts given in grams per liter in parentheses unless otherwise noted: glucose (80.0), KH$_2$(PO$_4$) (7.5), NaCl (3.0), (NH$_4$)$_2$HPO$_4$ (20.0), NH$_4$H$_2$PO$_4$ (20.0). myoinositol (0.3), yeast extract (Difco) (14.0), casein amino acids (enzymatic) (10.0), MgSO$_4$ (0.35), ZnSO$_4$ - (0.03), CuSO$_4$ (0.0045), FeNH$_4$(SO$_4$)$_2$ (0.06), thiamine (0.052), pyridoxine (0.015), calcium pantothenate (0.0075), biotin (0.01), SAG 471 antifoam (Union Carbide, Danbury, Connecticut) (0.25 ml/liter).

The yeast fermentation broth containing PUK was harvested after two to four days of fermentation, and cells were removed by centrifugation. The broth was diluted four-fold in cold deionized distilled water, adjusted to pH 6.5, and applied to a column containing CM-sepharose "fast-flow" (Pharmacia, Inc., Piscataway, NJ) equilibrated with buffer containing 50 mM sodium phosphate (pH 6.5) and 100 mM sodium chloride. After thorough washing of the column with equilibration buffer, the PUK was eluted from the column with a buffer consisting of 50 mM sodium phosphate (pH 6.5) and 600 mM sodium chloride. Following adjustment of the sodium chloride concentration to 1M and the pH to 7.0, the eluate, which contained an average of 52% of the initial PUK activity, was applied to a PABZ-Sepharose column (para-aminobenzamidine covalently linked to Sepharose; Collaborative Research, Inc., Bedford, MA) equilibrated in buffer containing 50 mM sodium phosphate (pH 7.0) and 1M sodium chloride. Purified PUK was eluted with a buffer consisting of 100 mM sodium acetate (pH 4.8) and 100 mM sodium chloride.

The purified PUK(A302) exhibited a specific activity virtually identical to that of natural PUK from TCL-598 cells, about 100,000 to 120,000 IU/mg, when measured in the chromogenic substrate assay with pyro-GLU-GLY-ARG-paranitroanilide (S2444; Kabi Vitrum, Stockholm, Sweden) following plasmin activation according to the method of Kohno et al.(1984, supra). Both natural PUK and PUK(A302) exhibited low specific activator (i.e.,< 1000 IU/mg) in the same assay without prior plasmin treatment. Analysis of the purified PUK(A302) on polyacrylamide gels containing SDS before and after reduction with 2-mercaptoethanol revealed that the protein migrates with an apparent molecular weight of about 47,000, consistent with the absence of carbohydrate, and that it is virtually all a single polypeptide chain even after reduction.

EXAMPLE 3

In another example of this invention, an active, non-glycosylated modified PUK having alanine at position 304 in place of threonine was produced in the yeast Saccharomyces cerevisiae. First, a modified PUK cDNA gene containing an alanine codon in place of the naturally occurring threonine codon at position 304 in the carbohydrate receiving region was constructed on a yeast-E. coli shuttle vector. DNA of plasmid pCGS715 (supra) was cut to completion with restriction endonuclease XbaI and partially with EcoRI (desired site is between codons 301 and 302 in the PUK cDNA), and the resulting approximately 4.81 kb DNA fragment was isolated by agarose gel electrophoresis. Another aliquot of pCGS715 DNA was cut completely with restriction endonucleases BglII (simply to facilitate later isolation of the desired fragment) and with XbaI and then partially with PvuII (desired site is between codons 311 and 312 of the PUK cDNA). The resulting approximately 5.70 kb DNA fragment was isolated by agarose gel electrophoresis. These two isolated fragments were ligated together with T4 DNA ligase in the presence of two annealed synthetic oligodeoxynucleotides providing EcoRI and PvuII cohesive ends, an alanine codon at position 304, a new AvaI restriction site (C!CCGAG) and having the following sequence.

9

```
            302 303 304 305 306 307 308 309 310 311
            asn ser ala asp tyr leu tyr pro glu gln
        5'  AAT TCT GCA GAC TAT CTC TAT CCC GAG CAG      3'
        3'      GA  CGT CTG ATA GAG ATA GGG CTC GTC      5'
```

The ligation mixture was used to transform competent HB101 E. coli cells to ampicillin resistance, and a transformed colony carrying the desired plasmid pCGS778 was identified by restriction enzyme analysis of plasmid DNA isolated from several transformant colonies. Plasmid pCGS778 contains a yeast TPI-promoted PUK cDNA having an alanine codon at position 304 and fused in frame to DNA encoding the invertase secretion signal. The identity of plasmid pCGS778 was verified by dideoxy sequencing of the PUK DNA in the region of the codon change and by the presence of the new Aval restriction site.

The following methods are accomplished as described in EXAMPLE 1 for plasmid pCGS721. DNA of plasmid pCGS778 was used to transform yeast strain CGY1585 to uracil prototrophy by standard methods known in the art. A transformant colony is picked and grown in SM-II semi-synthetic medium (same as SM-IV, supra, except yeast extract is reduced to 3.75 g/liter and casein amino acids are absent, but leucine is added 30 mg/liter). Secreted PUK(A304) in the unfractionated culture broth is analyzed in the fibrin plate assay (Brakman, 1967, supra) and by electrophoresis in a polyacrylamide gel containing SDS followed by immunoblot with rabbit anti-urokinase serum and $^{125}$I-labeled protein A. Secreted PUK(A304), like secreted PUK(A302) of EXAMPLE 1, is active in the fibrin plate assay, and exhibits an electrophoretic mobility consistent with the absence of any detectable carbohydrate.


## EXAMPLE 4


In another example of this invention, an active, non-glycosylated, modified PUK having glutamine at position 302 in place of asparagine was produced in the yeast Saccharomyces cerevisiae. First, a modified PUK cDNA gene containing a glutamine codon in place of the naturally occurring asparagine codon at position 302 was constructed on a yeast-E. coli shuttle vector. DNA of plasmid pCGS715 (supra) was cut partially with EcoRI (desired site is between codons 301 and 302 in the PUK cDNA), treated with the single stranded specific nuclease S1 to remove the overhanging termini, and following removal of the S1, was cut to completion with restriction endonuclease XbaI. The resulting approximately 4.81 kb DNA fragment was isolated by agarose gel electrophoresis. DNA of plasmid pCGS778 was cut completely with restriction endonuclease XbaI and then partially with Aval (desired site is in codon 309 of the PUK cDNA). The resulting approximately 5.70 kb DNA fragment was isolated by agarose gel electrophoresis. These two isolated fragments were ligated together with T4 DNA ligase in the presence of two annealed synthetic oligodeoxynucleotides providing an Aval cohesive end and a blunt end, a glutamine codon at position 302, and having the following sequence.

```
            302 303 304 305 306 307 308 309 310
            gln ser thr asp tyr leu tyr pro glu
        5'  CAG TCG ACA GAC TAT CTC TAT C        3'
        3'  GTC AGC TGT CTG ATA GAG ATA GGG CT   5'
```

The ligation mixture was used to transform competent HB101 E. coli cells to ampicillin resistance, and a transformed colony carrying the desired plasmid pCGS782 was identified by restriction enzyme analysis of plasmid DNA isolated from several transformant colonies. Plasmid pCGS782 contains a yeast TPI-promoted PUK cDNA having a glutamine codon at position 302 and fused in frame to DNA encoding the invertase secretion signal. The identity of plasmid pCGS782 was verified by dideoxy sequencing of the PUK DNA in the region of the codon change.

The following methods were accomplished precisely as described in EXAMPLE 1 for plasmid pCGS721. DNA of plasmid pCGS782 was used to transform yeast strain CGY1585 to uracil prototrophy by standard methods known in the art. A transformant colony was picked and grown in SM-II semi-synthetic medium. Secreted PUK(Q302) in the unfractionated culture broth was analyzed in the fibrin plate assay (Brakman, 1967, supra) and by electrophoresis in a polyacrylamide gel containing SDS followed by immunoblot with rabbit anit-urokinase serum and $^{125}$I-labeled protein A. Secreted PUK(Q302), like secreted PUK(A302) of EXAMPLE 1, was active in the fibrin plate assay, and exhibited an electrophoretic mobility consistent with the absence of any detectable carbohydrate.

In order to build a yeast strain secreting higher levels of PUK(Q302), the following procedure was followed. Two yeast integrating vectors were constructed based on plasmid Ylp5 (Struhl et al., 1979, supra). First, plasmid Ylp5 was modified as described in EXAMPLE 2 for the construction of plasmid pCGS740, except that plasmid pCGS782 DNA was used in place of pCGS721 as the source of PUK cDNA. As a result, the final plasmid pCGS815 carrys PGK-promoted PUK(Q302) fused to the invertase secretion signal instead of PGK-promoted PUK(A302) as is found on plasmid pCGS740. The second integrating vector consisted of Ylp5 carrying an approximately 4.1 kb DNA fragment from pCGS782 comprising the TPI promoter region, the invertase secretion signal codons, the PUK(Q302) cDNA and the SUC2 terminator region. This fragment was isolated following a partial EcoRI and complete SalI digest of pCGS782 DNA and was ligated to the approximately 4.9 kb DNA fragment isolated following EcoRI and SalI digestion of plasmid Ylp5. The resulting plasmid pCGS817 resembles pCGS815 except that the yeast promoter is from the TPI gene instead of the PGK gene and the selectable marker is URA3 instead of LEU2. Host strain CGY2022 (MAT alpha ura3-52 leu2-3,112 lys2 ssc1-1 ssc2-1) was transformed to uracil prototrophy with StuI-cut plasmid pCGS817 and to leucine prototrophy with ClaI-cut plasmid pCGS815 to yield the PUK(Q302)-secreting strain CGY2037 according to the method of Orr-Weaver et al. (1983; supra).

Strain CGY2037 was grown in SM-IV medium on a 10 to 20 liter scale exactly as described for strain CGY1892 in EXAMPLE 2 above. The PUK(Q302) was purified from the conditioned broth essentially as described for PUK(A302) in EXAMPLE 2 above except that S-Sepharose (Pharmacia, Piscataway, NJ) was substituted for CM-Sepharose as the first chromatography step and that step was conducted at pH 6.0 instead of 6.5.

The purified PUK(Q302) exhibited a specific activity virtually identical to that of natural PUK from TCL-598 cells, about 100,000 to 120,000 IU/mg, when measured in the chromogenic substrate assay with pyro-GLU-GLY-ARG-paranitroanilide (S2444; Kabi Vitrum, Stockholm, Sweden) following plasmin activation according to the method of Kohno et al.(1984, supra). Both natural PUK and PUK(Q302) exhibited low specific activity (i.e.,< 1000 IU/mg) in the same assay without prior plasmin treatment. Analysis of the purified PUK(Q302) on polyacrylamide gels containing SDS before and after reduction with 2-mercaptoethanol revealed that the protein migrates with an apparent molecular weight of about 47,000, consistent with the absence of carbohydrate, and that it is virtually all comprised of a single polypeptide chain even after reduction.

## EXAMPLE 5

In another example of this invention, an active, non-glycosylated, modified PUK having valine at position 304 in place of threonine was produced in the yeast Saccharomyces cerevisiae. First, a modified PUK cDNA gene containing a valine codon in place of the naturally occurring threonine codon at position 304 was constructed on a yeast-E. coli shuttle vector. DNA of plasmid pCGS715 (supra) was cut to completion with restriction endonuclease XbaI, and partially with EcoRI (desired site is between codons 301 and 302 in the PUK cDNA), and the resulting approximately 4.81 kb DNA fragment was isolated by agarose gel electrophoresis. DNA of plasmid pCGS778 was cut completely with restriction endonuclease XbaI and then partially with AvaI (desired site is in codon 309 of the PUK cDNA). The resulting approximately 5.70 kb DNA fragment was isolated by agarose gel electrophoresis. These two isolated fragments were ligated together with T4 DNA ligase in the presence of two annealed synthetic oligodeoxynucleotides providing AvaI and EcoRI cohesive ends, a valine codon at position 304, and having the following sequence.

```
      302 303 304 305 306 307 308 309 310
      asn ser val asp tyr leu tyr pro glu
   5'  AAT TCT GTC GAC TAT CTC TAT C          3'
   3'      GA CAG CTG ATA GAG ATA GGG CT      5'
```

The ligation mixture was used to transform competent HB101 E. coli cells to ampicillin resistance, and a transformed colony carrying the desired plasmid pCGS779 was identified by restriction enzyme analysis of plasmid DNA isolated from several transformant colonies. Plasmid pCGS779 contains a yeast TPI-promoted PUK cDNA having a valine codon at position 304 and fused in frame to DNA encoding the invertase secretion signal. The identity of plasmid pCGS779 was verified by dideoxy sequencing of the PUK DNA in the region of the codon change.

The following methods are accomplished as described in EXAMPLE 1 for plasmid pCGS721. DNA of plasmid pCGS779 was used to transform yeast strain CGY1585 to uracil prototrophy by standard methods known in the art. A transformant colony is picked and grown in SM-II semi-synthetic medium. Secreted PUK(V304) in the unfractionated culture broth is analyzed in the fibrin plate assay (Brakman, 1967, *supra*) and by electrophoresis in a polyacrylamide gel containing SDS followed by immunoblot with rabbit anti-urokinase serum and $^{125}$I-labeled protein A. Secreted PUK(V304), like secreted PUK(A302) of EXAMPLE 1, is active in the fibrin plate assay, and exhibits an electrophoretic mobility consistent with the absence of any detectable carbohydrate

## EXAMPLE 6

In another example of this invention, an active, non-glycosylated, modified PUK having alanine at position 302 in place of asparagine, was produced by Chinese hamster ovary cells according to the following procedure. First, a modified PUK cDNA gene containing an alanine codon in place of the naturally occurring asparagine codon at position 302 was constructed on a mammalian pSV2-gpt vector as follows. The full-length cDNA encoding naturally occurring "wild-type" PUK was introduced into pSV2-gpt (Mulligen, R. and Berg, P., 1981, Proc. Nat'l. Acad. Sci. USA 78: 2072-2076). Briefly, the full length PUK cDNA was isolated from plasmid pCGE195 (*supra*) after restriction with HindIII followed by filling-out the ends with E. coli DNA polymerase I (Klenow fragment) and all four deoxynucleotide triphosphates. DNA of plasmid pSV2-gpt was cut with restriction endonucleases HindIII and BglII, and the ends were filled out with E. coli DNA polymerase (Klenow fragment) and all four deoxynucleotide triphosphates. The approximately 2.3 kb PUK encoding fragment from pCGE195 was ligated to the approximately 5.25 kb vector fragment from pSV2-gpt, and the ligation mixture was used to transform competent E. coli cells to ampicillin resistance. Cells carrying the desired plasmid pCGM16 (FIG. 5) were identified by restriction endonuclease digestion analysis. Plasmid PCGM16 carrys the full length PUK encoding cDNA inserted in pSV2-gpt (FIG. 5).

After transformation of mammalian cells and integration into the chromosomes, this vector is capable of replication in mammalian cells and directing the expression of the added PUK cDNA from the SV40 early promoter. The secretion of sc-uPA from CHO cells which had been transformed with pCGM16 was verified by analysis of conditioned medium in the fibrin plate assay (Brakman, 1967, *supra*) and by assaying it for amidolytic activity (Kohno et al. , 1984, *supra*).

DNA of plasmid pCGM16 was cut to completion with restriction endonucleases KpnI and BglII, and the resulting approximately 5.4 kb DNA fragment was isolated by agarose gel electrophoresis. Another aliquot of pCGM16 DNA was cut to completion with restriction endonucleases NcoI and KpnI, and the resulting approximately 0.79 kb DNA fragment was isolated by agarose gel electrophoresis. DNA of plasmid pCGS721 (*supra*) was cut to completion with restriction endonucleases BglII and NcoI, and the resulting approximately 1.3 kb DNA fragment was isolated following agarose gel electrophoretic separation. These three isolated DNA fragments were mixed and ligated together with T4 DNA ligase, and the ligation mixture was used to transform competent HB101 E. coli cells to ampicillin resistance. A transformed colony carrying the desired plasmid pCGM32 was identified by restriction endonuclease analysis of plasmid DNA from several transformants (pCGM32 lacks an EcoRI site because of the codon change).

Plasmid pCGM32 is mammalian vector pSV2-gpt carrying the PUK cDNA with asparagine codon 302 altered to a codon for alanine. After transformation of mammalian cells and integration into the chromosomes, this vector is capable of replication in mammalian cells and directing the expression of the added PUK cDNA from the SV40 early promoter. The secretion of PUK from CHO cells which had been transformed with pCGM32 was verified by analysis of conditioned medium in the fibrin plate assay (Brakman, 1967, *supra*) and by assaying it for amidolytic activity (Kohno et al., 1984, *supra*).

Plasmid pCGM32 was used in conjunction with a second plasmid supplying selectable marker to co-transform chinese hamster ovary cell line DG44 (Urlaub, G., Kas, E., Carothers, A. M., and Chasin, L.A., 1983, Cell 33: 405-412) which completely lacks the diploid DHFR locus. The co-transformed selectable plasmid was either pSV2-DHFR (Subramani, S., Mulligan, R., and Berg, P., 1981, Mol. Cell. Biol. 1: 854-864) or pdhfr2. 9 (Crouse, G. F., MeEwan, R.N., and Pearson, M.L., 1983, Mol. Cell. Biol. 3: 257-266). Co-transformation was accomplished by means of the calcium precipitation procedure of Graham, F.L. and van der Eb (1973, Virology 52: 456-467). Transformants were selected in Ham's F12 medium supplemented with 10% fetal bovine serum, but lacking glycine, hypoxanthine and thymidine.

To increase the plasminogen activator expression level, resulting co-transformants were carried through a step-wise gene amplification procedure which involves challenging the cells with increasing concentrations of methotrexate (MTX) (Kaufman, R. and Sharp, P., 1982, J. Mol. Biol 159: 601-621). Suitable high level plasminogen activator producing clones wereidentified by amidolytic assay of cultures of cells which were resistant to high levels of MTX. Clones were grown in medium consisting of 1:1 DME:F12 which lacks thymidine and hypoxanthine but is supplemented with 10% fetal bovine serum. Following growth in T-flasks and roller bottles. conditioned medium containing between 2 and 20 ug/ml of plasminogen activator washarvested.

The PUK(A302) was purified from the harvested medium by means of the following procedure. Conditioned medium was filtered through a 0.2 micron pleated capsule filter (Gelman, Ann Arbor, MI) to remove particulate material, titrated to pH7.2-pH7.4, if necessary, and applied to a column of anti-PUK-Sepharose equilibrated with a buffer consisting of 10 mM sodium phosphate (pH7. 4), 0.14 M sodium chloride, 10 KIU/ml Aprotinin (Sigma, St. Louis, MO).

Anti-PUK-Sepharose was made by coupling PUK-specific monoclonal antibody to CNBr-activated Sepharose (Pharmacia, Piscataway, NJ). The monoclonal antibody was prepared by fusion of PUK immunized mouse spleen cells with antibody-secreting myeloma cells according to published procedures (Oi, V. and Herzenberg, L., 1980, in "Selected Methods in Cellular Immunology", pp. 351-372, W.H.freeman & Co., San Francisco, CA). Analysis of the antibody showed that it has about 30 times higher affinity for PUK than tcu-PA. The coupling procedure was accomplished essentially according to standard methods (see "Affinity Chromatography: Principles and Methods, a Pharmacia publication; also see Axen, R., Porath, J., and Ernback, S., 1967, Nature 214: 1302-1304, and March, S.C., Parikh, I., and Cuatrecasas, P., 1974, Analytical Biochemistry 60: 149-152) except that 20 mg of purified monoclonal antibody was coupled per ml of gel. After the reaction, the coupled Sepharose was filtered to remove the coupling buffer containing unreacted protein. Remaining active groups on the cyanogen bromide-activated Sepharose were blocked by adding a solution of 1 M ethanolamine (in coupling buffer) and mixing the suspension at room temperature for two hours. The coupled Sepharose was filtered again and washed with a buffer containing 0.1 M sodium acetate (pH 4.0) and 1 M NaCl, and then washed with buffer containing 0.01 M sodium phosphate (pH 7.2) and 1 M NaCl for a total of three times. The coupled resin was washed again with 3 M sodium thiocynate dissolved in buffer containing 0.01 M sodium phosphate (pH7 2), 0.14 M NaCl, and finally it was washed with the same buffer lacking sodium thiocynate. The coupled Sepharose was then used as an affinity matrix for purification of PUK and variants.

After washing with the equilibration buffer, the column was developed with 50 mM glycine (pH2) to elute the protein which had been bound to the antibody-Sepharose. The eluate was diluted with one-fourth volume of 100 mM sodium acetate (pH5.3), 1 M sodium chloride, adjusted to a pH of 5.3, and applied to a column of p-aminobenzamidine-Sepharose (CRI, Bedford, MA) equilibrated with a buffer consisting of 20 mM sodium acetate (pH5. 3), 0.1 M sodium chloride. Protein flowing through the column wasmonitored by absorbancy at 280 nm, collected and concentrated to about 1 mg/ml for further analyses described below.

The purified PUK(A302) exhibited a specific activity virtually identical to that of natural PUK from TCL-598 cells, about 100,000 to 120,000 IU/mg, when measured in the chromogenic substrate assay with pyro-GLU-GLY-ARG-paranitroanilide (S2444; Kabi Vitrum, Stockholm, Sweden) following plasmin activation according to the method of Kohno et al.(1984, supra). Both natural PUK and PUK(A302) exhibited low specific activity (i.e.,< 1000 IU/mg) in the same assay without prior plasmin treatment. Analysis of the purified PUK(A302) on polyacrylamide gels containing SDS before and after reduction with 2-mercaptoethanol revealed that the protein migrates with an apparent molecular weight of about 47,000, consistent with the absence of carbohydrate, and that it is virtually all a single polypeptide chain even after reduction.


## EXAMPLE 7


In another example of this invention, an active, non-glycosylated, modified PUK having alanine at position 304 in place of threonine, was produced by Chinese hamster ovary cells according to the following procedure. First, a modified PUK cDNA gene containing an alanine codon in place of the naturally occurring threonine codon at position 304 was constructed on a mammalian pSV2-gpt vector as follows. DNA of plasmid pCGM16 was cut to completion with restriction endonucleases KpnI and BglII, and the resulting approximately 5.4 kb DNA fragment was isolated by agarose gel electrophoresis. Another aliquot of pCGM16 DNA was cut to completion with restriction endonucleases NcoI and KpnI, and the resulting approximately 0.79 kb DNA fragment was isolated by agarose gel electrophoresis. DNA of plasmid pCGS778 (supra) was cut to completion with restriction endonucleases BglII and NcoI, and the resulting

13

approximately 1.3 kb DNA fragment was isolated following agarose gel electrophoretic separation. These three isolated DNA fragments were mixed and ligated together with T4 DNA ligase, and the ligation mixture was used to transform competent HB101 E. coli cells to ampicillin resistance. A transformed colony carrying the desired plasmid pCGM58 was identified by restriction endonuclease analysis of plasmid DNA from several transformants and by determination of the DNA sequence in the appropriate region of the plasmid according to the dideoxy chain termination method.

Plasmid pCGM58 is mammalian vector pSV2-gpt carrying the PUK cDNA with threonine codon 304 altered to a codon for alanine. After transformation of mammalian cells and integration into the chromosomes, this vector is capable of replication in mammalian cells and directing the expression of the added PUK cDNA from the SV40 early promoter. The secretion of PUK from CHO cells which had been transformed with pCGM58 was verified by analysis of conditioned medium in the fibrin plate assay (Brakman, 1967, supra) and by assaying it for amidolytic activity (Kohno et al., 1984, supra). The size of PUK(A304) as measured by separation on polyacrylamide gels containing SDS followed by immunoblot analysis with rabbit anti-urokinase serum and $^{125}$I-labeled protein A is consistent with the absence of any carbohydrate.


### EXAMPLE 8


In another example of this invention, an active, non-glycosylated, modified PUK having valine at position 304 in place of threonine, was produced by Chinese hamster ovary cells according to the following procedure. First, a modified PUK cDNA gene containing a valine codon in place of the naturally occurring threonine codon at position 304 was constructed on a mammalian pSV2-gpt vector as follows. DNA of plasmid pCGM16 was cut to completion with restriction endonucleases KpnI and BglII, and the resulting approximately 5.4 kb DNA fragment was isolated by agarose gel electrophoresis. Another aliquot of pCGM16 DNA was cut to completion with restriction endonucleases NcoI and KpnI, and the resulting approximately 0.79 kb DNA fragment was isolated by agarose gel electrophoresis. DNA of plasmid pCGS779 (supra) was cut to completion with restriction endonucleases BglII and NcoI, and the resulting approximately 1.3 kb DNA fragment was isolated following agarose gel electrophoretic separation. These three isolated DNA fragments were mixed and ligated together with T4 DNA ligase, and the ligation mixture was used to transform competent HB101 E. coli cells to ampicillin resistance. A transformed colony carrying the desired plasmid pCGM59 was identified by restriction endonuclease analysis of plasmid DNA from several transformants and by determination of the DNA sequence of the appropriate region of the plasmid by the dideoxy chain terminating method.

Plasmid pCGM59 is mammalian vector pSV2-gpt carrying the PUK cDNA with threonine codon 304 altered to a codon for valine. After transformation of mammalian cells and integration into the chromosomes, this vector is capable of replication in mammalian cells and directing the expression of the added PUK cDNA from the SV40 early promoter. The secretion of PUK from CHO cells which had been transformed with pCGM59 was verified by analysis of conditioned medium in the fibrin plate assay (Brakman, 1967, supra) and by assaying it for amidolytic activity (Kohno et al., 1984, supra). The size of PUK(V304) as measured by separation on polyacrylamide gels containing SDS followed by immunoblot analysis with rabbit anti-urokinase serum and $^{125}$I-labeled protein A is consistent with the absence of any carbohydrate.


### EXAMPLE 9


In another example of this invention, an active, non-glycosylated, modified PUK having glutamine at position 302 in place of asparagine was produced by Chinese hamster ovary cells according to the following procedure. First, a modified PUK cDNA gene containing a glutamine codon in place of the naturally occurring asparagine codon at position 302 was constructed on a mammalian pSV2-gpt vector as follows. DNA of plasmid pCGM16 was cut to completion with restriction endonucleases KpnI and BglII, and the resulting approximately 5. 4 kb DNA fragment was isolated by agarose gel electrophoresis. Another aliquot of pCGM16 DNA was cut to completion with restriction endonucleases NcoI and KpnI, and the resulting approximately 0. 79 kb DNA fragment was isolated by agarose gel electrophoresis. DNA of plasmid pCGS782 (supra) was cut to completion with restriction endonucleases BglII and NcoI, and the resulting approximately 1.3 kb DNA fragment was isolated following agarose gel electrophoretic separation

These three isolated DNA fragments were mixed and ligated together with T4 DNA ligase, and the ligation mixture was used to transform competent HB101 E. coli cells to ampicillin resistance. A transformed colony carrying the desired plasmid pCGM60 was identified by restriction endonuclease analysis of plasmid DNA from several transformants and by determination of the DNA sequence of the appropriate region of the plasmid by the dideoxy chain terminating method.

Plasmid pCGM60 is mammalian vector pSV2-gpt carrying the PUK cDNA with asparagine codon 302 altered to a codon for glutamine. After transformation of mammalian cells and integration into the chromosomes, this vector is capable of replication in mammalian cells and directing the expression of the added PUK cDNA from the SV40 early promoter. The secretion of PUK from CHO cells which had been transformed with pCGM60 was verified by analysis of conditioned medium in the fibrin plate assay (Brakman, 1967, supra) and by assaying it for amidolytic activity (Kohno et al., 1984. supra). The size of PUK(Q302) as measured by separation in polyacrylamide gels containing SDS followed by immunoblot analysis with rabbit anti-urokinase serum and $^{125}$I-labeled protein A is consistent with the absence of any carbohydrate.

## EXAMPLE 10

Purified mutant plasminogen activators PUK(A302), derived from yeast cells (EXAMPLE 2) or derived from CHO cells (EXAMPLE 6), and PUK(Q302) derived from yeast cells (EXAMPLE 4) were examined in the in vitro fibrin clot lysis model of Gurewich, V., Pannell, R., Louie, S., Kelley, P., Suddith, R.L., and Greenlee, R. (1983, J. Clin. Invest. 73: 1731-1739). Briefly, aliquots of citrated human plasma containing 1.5 uCi IBRIN ([125]-I-labeled fibrin; Amersham Corp, Arlington Heights, IL) were supplemented with 20 mM calcium chloride and 10 ul thromboplastin (T-0263; Sigma, St. Louis, MO) and incubated in 5 mm(i.d.) glass tubes for four hours at 37°C. The clots were removed from the glass tubes and bathed in 2.5 ml pooled human plasma containing the mutant plasminogen activators or purified wild-type PUK as a control at concentrations of about 10 to 50 nM. Potency wasjudged by the time and dose dependence of clot lysis; fibrin specificity was determined by measurement of residual plasma fibrinogen and alpha-2-antiplasmin levels by means of standard assays (Clauss, A., 1957, Acta. Hematol. 17: 237-246; Vermylen, C., deVreker, R.A., and Verstraete, M., 1963, Clinica Chemica Acta 8: 418-424; Edy, J., DeCook, F., and Collen, D., 1976, Thromb. Res. 8: 513-518). As shown in Table 1, the mutant plasminogen activators exhibited potency and fibrin specificity equal to that of wild-type PUK isolated from TCL-598 conditioned medium (Kohno et al., 1984, supra).

In contrast, when unmodified PUK (ie., having asparagine at position 302) was secreted from yeast cells and isolated from yeast culture broth, it was heavily glycosylated at asparagine 302 with mannose residues as is characteristic of many secreted yeast glycoproteins such as invertase and acid phosphatase. When this heavily glycosylated PUK was tested in the in vitro model system, it failed to lyse clots efficiently when administered at the same doses as non-glycosylated PUK(A302) or PUK(Q302)

### TABLE 1

| PUK | Dose (nM) | Lysis | Fibrinogen | Antiplasmin |
|---|---|---|---|---|
|  |  | (% after 4 hr) | (% remaining at 4 hr) | |
| WT | 25 | 70 | 70 | 40 |
| A302 | 25 | 100 | 70 | 52 |
| Q302 | 25 | 90 | NA | NA |
| NA = not available at this time. | | | | |

secreted from yeast. Therefore, glycosylation of PUK results in a heterogeneously sized PUK molecule but also one which is apparently less active at lysing fibrin clots in vitro.

## EXAMPLE 11

Purified mutant plasminogen activators PUK(A304) and PUK(V304) derived from yeast cells (EXAMPLES 3 and 5, respectively) or derived from CHO cells (EXAMPLES 7 and 8, respectively) are examined in the in vitro fibrin clot lysis model of Gurewich, V., Pannell, R., Louie, S., Kelley, P., Suddith, R.L., and Greenlee, R. (1983, J. Clin. Invest. 73: 1731-1739). Briefly, aliquots of citrated human plasma containing 1.5 uCi IBRIN ($^{125}$I- labeled fibrin; Amersham Corp, Arlington Heights, IL) are supplemented with 20 mM calcium chloride and 10 ul thromboplastin (T-0263; Sigma, St. Louis, MO) and incubated in 5 mm- (i.d.) glass tubes for four hours at 37°C. The clots are removed from the glass tubes and bathed in 2.5 ml pooled human plasma containing the mutant plasminogen activators or purified wild-type PUK as a control at concentrations of about 10 to 50 nM. Potency is judged by the time and dose dependence of clot lysis; fibrin specificity is determined by measurement of residual plasma fibrinogen and alpha-2-antiplasmin levels by means of standard assays (Clauss. A., 1957, Acta. Hematol. 17: 237-246; Vermylen. C., deVreker, R.A., and Verstraete, M., 1963, Clinica Chemica Acta 8: 418-424; Edy, J., DeCook, F., and Collen, D., 1976, Thromb. Res. 8: 513-518). The mutant plasminogen activators exhibit potency (% clot lysis) and fibrin specificity (% residual fibrinogen and alpha-2-antiplasmin) equal to that of wild-type PUK.

## EXAMPLE 12

The purified mutant plasminogen activators PUK(A302) and PUK(Q302) of EXAMPLES 2 and 4, respectively, were examined for potency and specificity during clot lysis in the rabbit jugular venous thrombosis model according to the protocol of Collen, D., Stassen, J.M., and Verstraete. M. (1983, J. Clin. Invest. 71: 368-376). As shown in Table 2, the mutant plasminogen activators exhibited potency (% lysis) and fibrin specificity (% residual fibrinogen and alpha-2-antiplasmin) approximately equal to that of wild-type PUK having asparagine at position 302 and secreted by the human kidney cell line TCL-598 (Kohno, T., et al. 1984, supra). The fibrin specificity of both PUK(A302) and PUK(Q302) were superior to those observed with plasmin-cleaved two chain urokinase (UK in the Table below) which is known to exhibit poor fibrin specificity (Stump, D.C., Stassen, J.M., Demarsin, E., and Collen, D., 1987, Blood 69: 592-596).

TABLE 2

| PUK | Dose (mg/kg) | No. | Lysis | Fibrinogen | Antiplasmin |
|---|---|---|---|---|---|
| | | | (% at 4.5 hr) | (% Remaining at 4.5 hr) | |
| None | 0 | 7 | 9 + - 0.8 | 90 +/- 2.1 | 89 +/- 2.7 |
| WT | 0.5 | 3 | 18 +/- 2.5 | 66 +/- 7.8 | 68 +/- 11 |
| | 1.0 | 3 | 33 +/- 3.6 | 56 +/- 40 | 78 +/- 1.4 |
| | 2.0 | 3 | 42 +/- 2.6 | 51 +/- 8.1 | 37 +/- 15.7 |
| A302 | 0.5 | 3 | 17 +- 0.7 | 66 +/- 28 | 61 +/- 37 |
| | 1.0 | 4 | 14 +/- 1.4 | 91 +/- 7.6 | 82 +/- 11 |
| | 2.0 | 3 | 21 +/- 1.0 | 82 +/- 11 | 67 +/- 22 |
| Q302 | 0.5 | 2 | 19 +- 0.5 | NA | NA |
| | 1.0 | 3 | 27 +/- 2.1 | NA | NA |
| | 2.0 | 4 | 38 +/- 11.4 | 51 +/- 10 | 35 +/- 1.5 |
| UK | 2.0 | 3 | 32 +/- 0.9 | 8 +/- 2.1 | 14 +/- 2.3 |
| NA = Not Available at this time. | | | | | |

EXAMPLE 13

The purified non-glycosylated plasminogen activator PUK(A302) of EXAMPLE 2 was examined for potency and specificity during clot lysis in the dog arterial thrombosis model of Van de Werf, F. , Jang, I.K. and Collen, D. (1987, J. Cardiovascular Pharmacol. 9: 91-93) except that heparin was administered directly after reperfusion of the blocked artery was observed angiographically. PUK(A302) showed potency (numbers of animals reperfused and times of reperfusion) and fibrin specificity (residual alpha-2-anitplasmin and fibrinogen) equivalent to those of wild-type natural PUK having asparagine at position 302 and secreted and glycosylated by CHO cells. Results are shown in Table 3.

The preceding examples can be repeated with similar success by substituting for prourokinase any of the other plasminogen activator molecules which contain an asparagine-linked carbohydrate receiving region, the site for N-linked glycosylation. As described above, an amino acid of the carbohydrate receiving region is removed and substituted with one or more amino acids. Amino acid substitution can occur at the asparagine and/or threonine (serine) sites and the substitution can be made with any one or more of the known amino acids. Similarly, amino acid modification mutants can arise with deletion or

TABLE 3

| PUK | Dose | # Reperfused per Total # Treated | Times of Reperfusion | Residual Antiplasmin | Residual Fibrinogen |
|------|---------|-----------------|-------------------|------------------|------------------|
|  | mg/kg/hr |  | (minutes) | (Avg % at 2 hr) | |
| None | 0 | 0/2 | >120 | 105 | 90 |
| WT | 1.0 | 5/6 | 25,25,40,15,75 | 52 | 72 |
| A302 | 1.0 | 3/3 | 30,15,15 | 51 | 70 |

insertion of amino acid residues which render the plasminogen activator molecule incapable of receiving N-linked carbohydrate residues. In addition, combinations of insertions, deletions or substitutions can be employed.

The modification of the carbohydrate receiving region can be carried out by any of the known recombinant DNA techniques or by chemical means with various agents such as those which selectively alter specific amino acids.

While examples of amino acid substitution have been described, deletion or insertion or other substitution in the asparagine-X-threonine/serine tripeptide can also be used to prevent addition of carbohydrate moities so long as no serine or threonine directly follows the tripeptide when a deletion is used. For example, either threonine, serine or asparagine in the tripeptide of PUK can be deleted. Further, insertion of amino acid residues between the asparagine and threonine of the asparagine-X-threonine/serine tripeptide can be used; however, if X is serine or threonine then the insertion must be between X and threonine and the insertion cannot itself be a serine or threonine in order to prevent addition of carbohydrate moities.

The amino acid modified plasminogen activator provides a nonglycosylated form which can be particularly effective in dissolving blood clots without eliciting the allergic reactions which are possible for some of the previously known plasminogen activators. The said modified plasminogen activator is of therapeutic value.

The nonglycosylated plasminogen activator in accordance with this invention can be of various types as previously described. For example, streptokinase, tissue plasminogen activator, prourokinase and the like can be treated in accordance with the methods of this invention to convert them into nonglycosylated forms. These products can be secreted through cell walls using known DNA cloning, expression and secretion procedures or other techniques. The cells used can be any of the host cells known in the art.

The nonglycosylated plasminogen activators can be used as dosage rates in vivo as previously known in the art. In some cases where activities may be increased, dosages are decreased. For example, when using nonglycosylated prourokinase in man and animals, it is preferred to use such dosage rates of from about 50 to about 150 milligrams for a man averaging 70 kilograms. In some cases, it is known to combine more than one plasminogen activator, as for example, use of mixtures of tissue plasminogen activator and prourokinase or mixtures of prourokinase and urokinase in administrations to blood in the body as by injection into the blood stream. Similarly, nonglycosylated forms of each can be used in known combinations according to the literature.

## Claims

1. A nonglycosylated secreted, plasminogen activator.

2. A plasminogen activator as claimed in claim 1 comprising an amino acid sequence which provides at least one site for glycosylation, characterised in that said sequence is modified to prevent addition of carbohydrate moieties at said site.

3. A plasminogen activator as claimed in claim 2 characterised in that said site or sites is modified to prevent N-linked carbohydrate addition.

4. A plasminogen activator as claimed in claim 3 characterised in that said amino acid sequence comprises an original tripeptide sequence asparagine-X-threonine/serine modified to prevent glycosylation.

5. A plasminogen activator as claimed in claim 4 characterised in that said plasminogen activator has been modified by mutagenesis.

6. A plasminogen activator as claimed in claim 5 characterised by comprising an amino acid substitution, an amino acid delection, or an amino acid insertion.

7. A plasminogen activator as claimed in claim 4 characterised by further comprising chemical modification.

8. A plasminogen activator in accordance with claim 2 characterised in that said one site is an asparagine 302-serine-303 threonine 304 tripeptide site, modified to prevent glycosylation.

9. A plasminogen activator in accordance with claim 8 characterised in that said asparagine is replaced by alanine or glutamine.

10. A plasminogen activator in accordance with claim 8 characterised in that said threonine is replaced by valine or alanine.

11. A method of producing nonglycosylated plasminogen activator from plasmainogen activator having at least one tripeptide sequence asparagine-X-threonine/serine, which is an N-linked carbohydrate receiving region, without substantially decreasing said plasminogen activator's function to activate plasminogen in initiating blood clot lysis, characterised by comprising treating said plasminogen activator so as to modify said N-linked carbohydrate receiving region.

12. A method in accordance with claim 11 characterised in that said plasminogen activator is modified by substitution, insertion, or deletion.

13. A composition characterised by comprising a therapeutically effective concentration of a non-glycosylated plasminogen activator as claimed in any of claims 1-10 in admixture with a pharmacologically acceptable excipient

14. A method of treating the body by adding a nonglycosylated plasminogen activator to blood within the body at a dosage level sufficient to dissolve clots without causing unwanted change to the body.

15. A plasminogen activator in accordance with any of claims 1-10 characterised in that said plasminogen activator is either a modified tissue-type plasminogen activator, a modified urokinase plasminogen activator, a modified prourokinase plasminogen activator, or a modified tissue plasminogen activator.

```
TCCCCGCAGCGCCGTCGCGCCCTCCTGCCGCAGGCCA
CCGAGGCCGCCGCCGTCTAGCGCCCCGACCTCGCCACC
```

-20
```
Met Arg Ala Leu Leu Ala Arg Leu Leu
ATG AGA GCC CTG CTG GCG CGC CTG CTT
        -10
Leu Cys Val Leu Val Val Ser Asp Ser
CTC TGC GTC CTG GTC GTG AGC GAC TCC
        1           ↑
Lys Gly Ser Asn Glu Leu His Gln Val
AAA GGC AGC AAT GAA CTT CAT CAA GTT
        10
Pro Ser Asn Cys Asp Cys Leu Asn Gly
CCA TCG AAC TGT GAC TGT CTA AAT GGA
            20
Gly Thr Cys Val Ser Asn Lys Tyr Phe
GGA ACA TGT GTG TCC AAC AAG TAC TTC
            30
Ser Asn Ile His Trp Cys Asn Cys Pro
TCC AAC ATT CAC TGG TGC AAC TGC CCA
                40
Lys Lys Phe Gly Gly Gln His Cys Glu
AAG AAA TTC GGA GGG CAG CAC TGT GAA
                    50
Ile Asp Lys Ser Lys Thr Cys Tyr Glu
ATA GAT AAG TCA AAA ACC TGC TAT GAG
                        60
Gly Asn Gly His Phe Tyr Arg Gly Lys
GGG AAT GGT CAC TTT TAC CGA GGA AAG
                            70
Ala Ser Thr Asp Thr Met Gly Arg Pro
GCC AGC ACT GAC ACC ATG GGC CGG CCC
```

FIG. 1

```
Cys  Leu  Pro  Trp  Asn  Ser  Ala  Thr  Val
TGC  CTG  CCC  TGG  AAC  TCT  GCC  ACT  GTC
80

Leu  Gln  Gln  Thr  Tyr  His  Ala  His  Arg
CTT  CAG  CAA  ACG  TAC  CAT  GCC  CAC  AGA
          90

Ser  Asp  Ala  Leu  Gln  Leu  Gly  Leu  Gly
TCT  GAT  GCT  CTT  CAG  CTG  GGC  CTG  GGG
               100

Lys  His  Asn  Tyr  Cys  Arg  Asn  Pro  Asp
AAA  CAT  AAT  TAC  TGC  AGG  AAC  CCA  GAC
                    110

Asn  Arg  Arg  Arg  Pro  Trp  Cys  Tyr  Val
AAC  CGG  AGG  CGA  CCC  TGG  TGC  TAT  GTG
                         120

Gln  Val  Gly  Leu  Lys  Pro  Leu  Val  Gln
CAG  GTG  GGC  CTA  AAG  CCG  CTT  GTC  CAA
                         130

Glu  Cys  Met  Val  His  Asp  Cys  Ala  Asp
GAG  TGC  ATG  GTG  CAT  GAC  TGC  GCA  GAT
                              140

Gly  Lys  Lys  Pro  Ser  Ser  Pro  Pro  Glu
GGA  AAA  AAG  CCC  TCC  TCT  CCT  CCA  GAA
                                   150

Glu  Leu  Lys  Phe  Gln  Cys  Gly  Gln  Lys
GAA  TTA  AAA  TTT  CAG  TGT  GGC  CAA  AAG
                                        160

Thr  Leu  Arg  Pro  Arg  Phe  Lys  Ile  Ile
ACT  CTG  AGG  CCC  CGC  TTT  AAG  ATT  ATT

Gly  Gly  Glu  Phe  Thr  Thr  Ile  Glu  Asn
GGG  GGA  GAA  TTC  ACC  ACC  ATC  GAG  AAC
```

FIG. 1 (Continued)

170
Gln Pro Trp Phe Ala Ala Ile Tyr Arg
CAG CCC TGG TTT GCG GCC ATC TAC AGG
        180
Arg His Arg Gly Gly Ser Val Thr Tyr
AGG CAC CGG GGG GGC TCT GTC ACC TAC
        190
Val Cys Gly Gly Ser Leu Ile Ser Pro
GTG TGT GGA GGC AGC CTC ATC AGC CCT
        200
Cys Trp Val Ile Ser Ala Thr His Cys
TGC TGG GTG ATC AGC GCC ACA CAC TGC
        210
Phe Ile Asp Tyr Pro Lys Lys Glu Asp
TTC ATT GAT TAC CCA AAG AAG GAG GAC
        220
Tyr Ile Val Tyr Leu Gly Arg Ser Arg
TAC ATC GTC TAC CTG GGT CGC TCA AGG
        230
Leu Asn Ser Asn Thr Gln Gly Glu Met
CTT AAC TCC AAC ACG CAA GGG GAG ATG
        240
Lys Phe Glu Val Glu Asn Leu Ile Leu
AAG TTT GAG GTG GAA AAC CTC ATC CTA
        250
His Lys Asp Tyr Ser Ala Asp Thr Leu
CAC AAG GAC TAC AGC GCT GAC ACG CTT

Ala His His Asn Asp Ile Ala Leu Leu
GCT CAC CAC AAC GAC ATT GCC TTG CTG
260
Lys Ile Arg Ser Lys Glu Gly Arg Cys
AAG ATC CGT TCC AAG GAG GGC AGG TGT

FIG. 1 (Continued)

270
Ala Gln Pro Ser Arg Thr Ile Gln Thr
GCG CAG CCA TCC CGG ACT ATA CAG ACC
            280
Ile Cys Leu Pro Ser Met Tyr Asn Asp
ATC TGC CTG CCC TCG ATG TAT AAC GAT
            290
Pro Gln Phe Gly Thr Ser Cys Glu Ile
CCC CAG TTT GGC ACA AGC TGT GAG ATC
            300
Thr Gly Phe Gly Lys Glu Asn Ser Thr
ACT GGC TTT GGA AAA GAG AAT TCT ACC
            310
Asp Tyr Leu Tyr Pro Glu Gln Leu Lys
GAC TAT CTC TAT CCG GAG CAG CTG AAA
            320
ATG ACT GTT GTG AAG CTG ATT TCC CAC
Met Thr Val Val Lys Leu Ile Ser His
            330
Arg Glu Cys Gln Gln Pro His Tyr Tyr
CGG GAG TGT CAG CAG CCC CAC TAC TAC
            340
GGC TCT GAA GTC ACC ACC AAA ATG CTA
Gly Ser Glu Val Thr Thr Lys Met Leu

Cys Ala Ala Asp Pro Gln Trp Lys Thr
TGT GCT GCT GAC CCC CAA TGG AAA ACA
350
GAT TCC TGC CAG GGA GAC TCA GGG GGA
Asp Ser Cys Gln Gly Asp  Ser Gly Gly
            360
Pro Leu Val Cys Ser Leu Gln Gly Arg
CCC CTC GTC TGT TCC CTC CAA GGC CGC

FIG. 1 (Continued)

```
        370
ATG ACT TTG ACT GGA ATT GTG AGC TGG
Met Thr Leu Thr Gly Ile Val Ser Trp
                380
Gly Arg Gly Cys Ala Leu Lys Asp Lys
GGC CGT GGA TGT GCC CTG AAG GAC AAG
                390
Pro Gly Val Tyr Thr Arg Val Ser His
CCA GGC GTC TAC ACG AGA GTC TCA CAC
                400
Phe Leu Pro Trp Ile Arg Ser His Thr
TTC TTA CCC TGG ATC CGC AGT CAC ACC
                410
AAG GAA GAG AAT GGC CTG GCC CTC TGA
Lys Glu Glu Asn Gly Leu Ala Leu ---
```

```
GGGTCCCCAGGGAGGAAACGGGCACCACCCGCTTTC
TTGCTGGTTGTCATTTTTGCAGTAGAGTCATCTCCA
TCAGCTGTAAGAAGAGACTGGGAAGATAGGCTCTGC
ACAGATGGATTTGCCTGTGGCACCACCAGGGTGAAC
GACAATAGCTTTACCCTCACGGATAGGCCTGGGTGC
TGGCTGCCCAGACCCTCTGGCCAGGATGGAGGGGGTG
GTCCTGACTCAACATGTTACTGACCAGCAACTTGTC
TTTTTCTGGACTGAAGCCTGCAGGAGTTAAAAAGGG
CAGGGCATCTCCTGTGCATGGGCTCGAAGGGAGAGC
CAGCTCCCCCGACCGGTGGGCATTTGTGAGGCCCAT
GGTTGAGAAATGAATAATTTCCCAATTAGGAAGTGT
AAGCAGCTGAGGTCTCTTGAGGGAGCTTAGCCAATG
TGGGAGCAGCGGTTTGGGGAGCAGAGACACTAACGA
CTTCAGGGCAGGGCTCTGATATTCCATGAATGTATC
AGGAAATATATGTGTGTGTATGTTTGCACACTTG
TTGTGTGGGCTGTGAGTGTAAGTGTGAGTAAGAGCT
GGTGTCTGATTGTTAAGTCTAAATATTTCCTTAAAC
```

FIG. 1 (Continued)

```
TGTGTGGACTGTGATGCCACACAGAGTGGTCTTTCT
GGAGAGGTTATAGGTCACTCCTGGGGCCTCTTGGGT
CCCCCACGTGACAGTGCCTGGGAATGTACTTATTCT
GCAGCATGACCTGTGACCAGCACTGTCTCAGTTTCA
CTTTCACATAGATGTCCCTTTCTTGGCCAGTTATCC
CTTCCTTTTAGCCTAGTTCATCCAATCCTCACTGGG
TGGGGTGAGGACCACTCCTTACACTGAATATTTATA
TTTCACTATTTTTATTTATATTTTTGTAATTTTAAA
TAAAAGTGATCAATAAAATGTGATTTTTCTGA
```

FIG. 1 (Continued)

Neu eingereicht / Newly filed
Nouvellement déposé

FIG. 2

FIG. 3

## FIG. 4

pCGS740

Ap$^r$

PPGK

INVERTASE SIGNAL

PUK(A302)

T

LEU2

pCGS732

Ap$^r$

Xho

Ty

PTPI

INVERTASE SIGNAL

PUK(A302)

T

URA3

Ty

Xho

## FIG. 5

pCGM16

EcoRI

FspI

amp$^r$

SV40 DNA

Kpnl

SV40 EARLY PROMOTER

P

Ncol

SS

BssHII

Ncol

BglII

FspI

EcoRI

PUK

FspI

EcoRI

Ncol